# EUROPÄISCHE PATENTANMELDUNG

(11) **EP 4 725 422 A1**
(43) Veröffentlichungstag der Anmeldung: **15.04.2026**
(21) Anmeldenummer: 25210000.3
(22) Anmeldetag: 21.10.2025
(51) Int. Cl.: A61B 6/03, A61B 6/00

(54) **MEDIZINISCHES BILDGEBUNGSSYSTEM MIT EINEM SCHIENENSYSTEM**

(30) Priorität: 27.11.2024 DE 102024211348
(71) Anmelder: Siemens Healthineers AG, 91301 Forchheim (DE)
(72) Erfinder: Fehre, Jens, 91301 Forchheim (DE); Garcia, Elmar, 91301 Forchheim (DE); Körber, Markus, 91301 Forchheim (DE)
(74) Vertreter: Siemens Healthineers Patent Attorneys

(57) **Zusammenfassung**

Die Erfindung betrifft ein medizinisches Bildgebungssystem.

Die Erfindung betrifft ein medizinisches Bildgebungssystem, aufweisend eine Computertomographie-Gantry, einen Fahrwagen, ein Schienensystem und ein Tragprofil,
- wobei die Computertomographie-Gantry mittels des Fahrwagens und des Schienensystems derart bewegbar gelagert ist, dass entlang des Schienensystems eine Translationsbewegung der Computertomographie-Gantry relativ zu einer Grundfläche ausführbar ist,
- wobei das Tragprofil in eine Vertiefung, die relativ zu der Grundfläche ausgebildet ist, formschlüssig aufgenommen ist,
- wobei das Tragprofil eine Schienen-Nut zur formschlüssigen Aufnahme einer Schiene des Schienensystems aufweist,
- wobei die Schiene des Schienensystems in die Schienen-Nut derart formschlüssig aufgenommen ist, dass die Schiene des Schienensystems in Bezug auf eine vertikale Richtung nicht über die Grundfläche hinausragt.

## Beschreibung

Die Erfindung betrifft ein medizinisches Bildgebungssystem.

Schienen von schienengeführten Medizingeräten werden oft mit Abdeckungen im Boden verbaut oder auf dem Boden aufbauend verbaut. Ersteres benötigt Abdeckungen gegenüber Flüssigkeiten, Staub o.ä. Letztere können aufgrund der Erhöhung relativ zu dem Bodenniveau als unangenehm wahrgenommen werden (insbesondere bei Erhöhungen bis 3 mm) oder als Stolperfalle wirken (insbesondere bei Erhöhungen > 3mm).

Als Stand der Technik ist hierbei die DE 10 2023 202 908 A1 zu nennen.

Die Erfindung hat die Aufgabe, eine Bewegung einer Computertomographie-Gantry zu ermöglichen, welche in Bezug auf Installation, Wartung und/oder Reinigbarkeit der beteiligten Komponenten verbessert ist.

Jeder Gegenstand eines unabhängigen Anspruchs löst diese Aufgabe. In den abhängigen Ansprüchen sind weitere vorteilhafte Aspekte der Erfindung berücksichtigt. Unabhängig vom grammatikalischen Geschlecht eines bestimmten Begriffes sind Personen mit männlicher, weiblicher oder anderer Geschlechteridentität mit umfasst.

Die Erfindung betrifft ein medizinisches Bildgebungssystem, aufweisend eine Computertomographie-Gantry, einen Fahrwagen, ein Schienensystem und ein Tragprofil,
- wobei die Computertomographie-Gantry mittels des Fahrwagens und des Schienensystems derart bewegbar gelagert ist, dass entlang des Schienensystems eine Translationsbewegung der Computertomographie-Gantry relativ zu einer Grundfläche ausführbar ist,
- wobei das Tragprofil in eine Vertiefung, die relativ zu der Grundfläche ausgebildet ist, formschlüssig aufgenommen ist,
- wobei das Tragprofil eine Schienen-Nut zur formschlüssigen Aufnahme einer Schiene des Schienensystems aufweist,
- wobei die Schiene des Schienensystems in die Schienen-Nut derart formschlüssig aufgenommen ist, dass die Schiene des Schienensystems in Bezug auf eine vertikale Richtung nicht über die Grundfläche hinausragt.

Die Schiene des Schienensystems ist somit nicht störend beim Drauftreten und benötigt keine Abdeckungen gegen Flüssigkeiten und/oder Staub, welche aufgrund der mechanischen Belastungen oft gewartet werden müssten. Ferner ist die offene Ausgestaltung der Schienen leichter zugänglich für Reinigungen.

Optional kann vorgesehen sein, dass jede zu der Grundfläche parallele Tangentialebene an die Schiene des Schienensystems nicht oberhalb der Grundfläche verläuft.

Optional kann vorgesehen sein, dass die Grundfläche einen ersten Grundflächenbereich und einen zweiten Grundflächenbereich aufweist,
- wobei der erste Grundflächenbereich und der zweite Grundflächenbereich zueinander komplanar sind,
- wobei sich die Vertiefung zwischen dem ersten Grundflächenbereich und dem zweiten Grundflächenbereich befindet,
- wobei die Schiene des Schienensystems in die Schienen-Nut derart formschlüssig aufgenommen ist, dass die Schiene des Schienensystems nicht über den ersten Grundflächenbereich hinausragt und dass die Schiene des Schienensystems nicht über den zweiten Grundflächenbereich hinausragt.

Insbesondere kann vorgesehen sein, dass sich die Vertiefung in Bezug auf eine Querrichtung, die zu einer Längsrichtung der Schiene des Schienensystems senkrecht und/oder zu der Grundfläche parallel ist, zwischen dem ersten Grundflächenbereich und dem zweiten Grundflächenbereich befindet. Beispielsweise können der erste Grundflächenbereich und der zweite Grundflächenbereich durch die Vertiefung voneinander getrennt sein.

Insbesondere kann vorgesehen sein, dass die zu der Grundfläche parallele Tangentialebene an den höchsten Punkt der Schiene des Schienensystems nicht oberhalb des ersten Grundflächenbereichs und/oder nicht oberhalb des zweiten Grundflächenbereichs verläuft. Insbesondere kann vorgesehen sein, dass die zu der Grundfläche parallele Tangentialebene an den höchsten Punkt der Schiene des Schienensystems zu dem ersten Grundflächenbereich und/oder zu dem zweiten Grundflächenbereich komplanar ist.

Optional kann vorgesehen sein, dass das Tragprofil eine Anschlussfläche aufweist, wobei das Tragprofil in die Vertiefung derart formschlüssig aufgenommen ist, dass die Anschlussfläche stufenlos, insbesondere komplanar, an die Grundfläche, insbesondere als eine Fortsetzung der Grundfläche, angefügt ist.

Die Fortsetzung der Grundfläche kann insbesondere stetig sein. Insbesondere kann vorgesehen sein, dass die zu der Grundfläche parallele Tangentialebene an den höchsten Punkt der Schiene des Schienensystems zu der Grundfläche und/oder zu der Anschlussfläche komplanar ist.

Optional kann vorgesehen sein, dass die Anschlussfläche einen ersten Anschlussflächenbereich und einen zweiten Anschlussflächenbereich aufweist,
- wobei sich die Schiene des Schienensystems zwischen dem ersten Anschlussflächenbereich und dem zweiten Anschlussflächenbereich befindet,
- wobei das Tragprofil in die Vertiefung derart formschlüssig aufgenommen ist, dass der erste Anschlussflächenbereich stufenlos, insbesondere komplanar, an den ersten Grundflächenbereich, insbesondere als eine Fortsetzung des ersten Grundflächenbereichs, angefügt ist und dass der zweite Anschlussflächenbereich stufenlos, insbesondere komplanar, an den zweiten Grundflächenbereich, insbesondere als eine Fortsetzung des zweiten Grundflächenbereichs, angefügt ist.

Die Fortsetzung des ersten Grundflächenbereichs kann insbesondere stetig sein. Die Fortsetzung des zweiten Grundflächenbereichs kann insbesondere stetig sein. Insbesondere kann vorgesehen sein, dass sich die die Schiene des Schienensystems in Bezug auf eine Querrichtung, die zu einer Längsrichtung der Schiene des Schienensystems senkrecht und/oder zu der Grundfläche parallel ist, zwischen dem ersten Anschlussflächenbereich und dem zweiten Anschlussflächenbereich befindet. Der erste Anschlussflächenbereich und der zweite Anschlussflächenbereich können beispielsweise zueinander komplanar sein.

Optional kann vorgesehen sein, dass sich die Grundfläche im Wesentlichen horizontal erstreckt,
- wobei die Schiene des Schienensystems in die Schienen-Nut derart formschlüssig aufgenommen ist, dass die Schiene des Schienensystems in Bezug auf die vertikale Richtung nicht über die Grundfläche hinausragt.

Insbesondere kann vorgesehen sein, dass die zu der Grundfläche parallele Tangentialebene an die Schiene des Schienensystems horizontal ist und/oder dass sich die Anschlussfläche im Wesentlichen horizontal erstreckt. Insbesondere kann vorgesehen sein, dass die Längsrichtung der Schiene des Schienensystems horizontal ist und/oder dass die Querrichtung, die zu der Längsrichtung der Schiene des Schienensystems senkrecht und/oder zu der Grundfläche parallel ist, horizontal ist. Insbesondere kann vorgesehen sein, dass der höchste Punkt der Schiene des Schienensystems in Bezug auf die vertikale Richtung nicht höher als die Grundfläche angeordnet ist.

Optional kann vorgesehen sein, dass die Schiene des Schienensystems eine erste Schiene des Schienensystems ist und parallel zu einer zweiten Schiene des Schienensystems angeordnet ist,
- wobei die Grundfläche im Wesentlichen parallel zu einer Schienenebene ist, wobei die Schienenebene durch die erste Schiene des Schienensystems und durch die zweite Schiene des Schienensystems verläuft.

Optional kann vorgesehen sein, dass die Schiene des Schienensystems in einer Querschnittsebene, die zu einer Längsrichtung der Schiene des Schienensystems senkrecht ist, ein Rundprofil, insbesondere ein kreisrundes Profil, aufweist.

Insbesondere kann vorgesehen sein, dass die Schiene des Schienensystems in einer Querschnittsebene, die zu einer Längsrichtung der Schiene des Schienensystems senkrecht ist, ein konvexes Profil, insbesondere ein kreisrundes Profil, aufweist.

Optional kann vorgesehen sein, dass die Schiene des Schienensystems in einer Querschnittsebene, die zu einer Längsrichtung der Schiene des Schienensystems senkrecht ist, ein verrundetes Rechteckprofil aufweist.

Optional kann vorgesehen sein, dass das Tragprofil im Bereich der Schienen-Nut ein profilseitiges Verbindungselement aufweist,
- wobei die Schiene des Schienensystems ein schienenseitiges Verbindungselement aufweist, das zu dem profilseitigen Verbindungselement korrespondierend ausgebildet ist,
- wobei die Schiene des Schienensystems durch einen Formschluss des profilseitigen Verbindungselements und des schienenseitigen Verbindungselements gegen eine Drehung um eine Längsachse der Schiene des Schienensystems relativ zu dem Tragprofil gesichert ist.

Die Erfindung betrifft ferner ein medizinisches Bildgebungssystem, aufweisend eine Computertomographie-Gantry, einen Fahrwagen, ein Schienensystem und ein Tragprofil,
- wobei die Computertomographie-Gantry mittels des Fahrwagens und des Schienensystems derart bewegbar gelagert ist, dass entlang des Schienensystems eine Translationsbewegung der Computertomographie-Gantry relativ zu einer Grundfläche ausführbar ist,
- wobei das Tragprofil in eine Vertiefung, die relativ zu der Grundfläche ausgebildet ist, formschlüssig aufgenommen ist,
- wobei das Tragprofil eine Schienen-Nut zur formschlüssigen Aufnahme einer Schiene des Schienensystems aufweist,
- wobei das Tragprofil im Bereich der Schienen-Nut ein profilseitiges Verbindungselement aufweist,
- wobei die Schiene des Schienensystems ein schienenseitiges Verbindungselement aufweist, das zu dem profilseitigen Verbindungselement korrespondierend ausgebildet ist,
- wobei die Schiene des Schienensystems durch einen Formschluss des profilseitigen Verbindungselements und des schienenseitigen Verbindungselements gegen eine Drehung um eine Längsachse der Schiene des Schienensystems relativ zu dem Tragprofil gesichert ist.

Optional kann vorgesehen sein, dass das profilseitige Verbindungselement stiftförmig in einer Richtung, die zu der Längsachse der Schiene des Schienensystems senkrecht ist, zu der Schiene des Schienensystems hin ragt,
- wobei das schienenseitige Verbindungselement eine Aussparung zur Aufnahme des profilseitigen Verbindungselements aufweist.

Optional kann vorgesehen sein, dass das schienenseitige Verbindungselement stiftförmig in einer Richtung, die zu der Längsachse der Schiene des Schienensystems senkrecht ist, von der Schiene des Schienensystems weg ragt,
- wobei das profilseitige Verbindungselement eine Aussparung zur Aufnahme des schienenseitigen Verbindungselements aufweist.

Optional kann vorgesehen sein, dass die Aussparung eine Bohrung, insbesondere in Form eines Kreislochs, ist.

Optional kann vorgesehen sein, dass die Aussparung ein Langloch ist und/oder sich parallel zu der Längsachse der Schiene des Schienensystems länglich erstreckt.

Insbesondere kann vorgesehen sein, dass die Schiene des Schienensystems in die Schienen-Nut formschlüssig aufgenommen werden kann, indem die Schiene des Schienensystem in die Schienen-Nut eingelegt wird, insbesondere durch ein Absenken der Schiene des Schienensystems relativ zu dem Tragprofil in einer zu der Längsachse der Schiene des Schienensystem senkrechten vertikalen Absenkrichtung eingelegt wird. Dazu kann das Tragprofil beispielsweise frei von Hinterschneidungen ausgebildet sein und/oder derart mehrteilig ausgebildet sein, dass sich Hinterschneidungen erst nach dem Einlegen der Schiene des Schienensystems in die Schienen-Nut und dem anschließenden Zusammenfügen des Tragprofils bilden.

Optional ist vorgesehen, dass der Fahrwagen und das Schienensystem dazu eingerichtet sind, eine Antriebskraft für die Translationsbewegung der Computertomographie-Gantry kraftschlüssig von dem Fahrwagen auf das Schienensystem zu übertragen, insbesondere durch Reibung zu übertragen.

Insbesondere kann vorgesehen sein, dass das Schienensystem relativ zu einer Grundfläche ruht und/oder relativ zu der Grundfläche fest verankert ist. Insbesondere kann vorgesehen sein, dass ein Untersuchungsobjekt relativ zu dem Schienensystem und/oder relativ zu der Grundfläche ruht. Das Schienensystem kann insbesondere eine Linearführung für den Fahrwagen bilden.

Beispielsweise kann das medizinische Bildgebungssystem einen Untersuchungstisch zur Lagerung des Untersuchungsobjekts aufweisen. Der Untersuchungstisch kann insbesondere relativ zu dem Schienensystem und/oder relativ zu der Grundfläche ruhen und/oder relativ zu dem Schienensystem und/oder relativ zu der Grundfläche fest verankert sein. Das Untersuchungsobjekt kann beispielsweise eine zu untersuchende Person, insbesondere ein Patient, sein und/oder auf dem Untersuchungstisch gelagert sein, insbesondere relativ zu dem Untersuchungstisch ruhend gelagert sein.

Die Translationsbewegung kann insbesondere relativ zu dem Schienensystem, relativ zu der Grundfläche, relativ zu dem Untersuchungstisch und/oder relativ zu dem Untersuchungsobjekt erfolgen. Die Translationsbewegung kann insbesondere im Wesentlichen horizontal sein. Die Grundfläche kann insbesondere im Wesentlichen horizontal sein. Die Grundfläche kann insbesondere ein Boden eines Untersuchungsraumes und/oder aus Beton hergestellt sein.

Die Computertomographie-Gantry kann beispielsweise einen Tragrahmen und einen relativ zu dem Tragrahmen drehbar gelagerten Rotor aufweisen, wobei die Strahlungsquelle und der Strahlungsdetektor an dem Rotor angeordnet sind. Optional kann die Computertomographie-Gantry einen relativ zu dem Tragrahmen kippbar gelagerten Kipprahmen aufweisen, wobei der Rotor an dem Kipprahmen angeordnet ist. Die Strahlungsquelle und der Strahlungsdetektor können für eine Aufnahme eines Projektionsdatensatzes von dem Untersuchungsobjekt zusammenwirken. Die Computertomographie-Gantry kann beispielsweise eine Öffnung aufweisen. Insbesondere können das Schienensystem, der Untersuchungstisch und die Öffnung derart zueinander angeordnet sein, dass durch die Translationsbewegung der Computertomographie-Gantry der Untersuchungstisch in die Öffnung eingeführt wird, insbesondere zusammen mit dem auf dem Untersuchungstisch gelagerten Untersuchungsobjekt in die Öffnung eingeführt wird.

Eine Ausführungsform sieht vor, dass zwischen dem Fahrwagen und dem Schienensystem ein Satz von Rad-Schiene-Rollkontakten ausgebildet ist, wobei der Fahrwagen und das Schienensystem dazu eingerichtet sind, die Antriebskraft für die Translationsbewegung der Computertomographie-Gantry mittels des Satzes von Rad-Schiene-Rollkontakten kraftschlüssig von dem Fahrwagen auf das Schienensystem zu übertragen.

Eine Ausführungsform sieht vor, dass der Satz von Rad-Schiene-Rollkontakten die gesamte Gewichtskraft des Fahrwagens und der Computertomographie-Gantry aufnimmt, wobei jeder Rad-Schiene-Rollkontakt, welcher in dem Satz von Rad-Schiene-Rollkontakten enthalten ist und zumindest einen Teil der gesamten Gewichtskraft des Fahrwagens und der Computertomographie-Gantry aufnimmt, zumindest einen Teil der Antriebskraft für die Translationsbewegung der Computertomographie-Gantry kraftschlüssig überträgt, insbesondere durch Reibung überträgt.

Insbesondere kann vorgesehen sein, dass der zumindest eine Teil der gesamten Gewichtskraft des Fahrwagens und der Computertomographie-Gantry nicht unwesentlich ist, beispielsweise größer als ein Zehntel der gesamten Gewichtskraft des Fahrwagens und der Computertomographie-Gantry ist. Insbesondere kann vorgesehen sein, dass der zumindest eine Teil der Antriebskraft für die Translationsbewegung der Computertomographie-Gantry nicht unwesentlich ist, beispielsweise größer als ein Zehntel der Antriebskraft für die Translationsbewegung der Computertomographie-Gantry ist.

Insbesondere kann für das medizinische Bildgebungssystem ausgeschlossen sein, dass es einen Rad-Schiene-Rollkontakt gibt, der zwar einen Teil der gesamten Gewichtskraft des Fahrwagens und der Computertomographie-Gantry aufnimmt, jedoch keinen Teil der Antriebskraft für die Translationsbewegung der Computertomographie-Gantry überträgt.

Die für den Antrieb zur Verfügung stehende Reibkraft hängt vom Reibwert und der Normalkraft ab, mit der das Reibrad belastet wird. Insbesondere wenn das Rad direkt angetrieben wird und die Reibung der Rad-Schiene-Rollkontakte ausgenutzt wird, kann die gesamte Gewichtskraft des Fahrwagens und der Computertomographie-Gantry als Normalkraft genutzt werden. Bei einem Reibrad kommt es zu einer Gewichtskraftaufteilung zwischen den Schienenrädern und dem Reibrad, so dass nur ein Teil der Gewichtskraft als Normalkraft zur Verfügung steht.

Eine Ausführungsform sieht vor, dass das Schienensystem einen Satz von Schienen aufweist, wobei der Fahrwagen einen Satz von Rädern aufweist, wobei der Satz von Rädern auf dem Satz von Schienen rollend angeordnet ist. Der Satz von Schienen kann beispielsweise die Schiene des Schienensystems aufweisen. Der Satz von Schienen kann beispielsweise die erste Schiene des Schienensystems und/oder die zweite Schiene des Schienensystems aufweisen.

Insbesondere kann vorgesehen sein, dass der Satz von Schienen und der Satz von Rädern den Satz von Rad-Schiene-Rollkontakten bilden. Insbesondere kann vorgesehen sein, dass jede Schiene des Satzes von Schienen eine Rundschiene ist und/oder dass jedes Rad des Satzes von Rädern eine konkave Rolle und/oder zum Abrollen auf einer Rundschiene ausgebildet ist. Die Schienen und/oder die Räder können beispielsweise aus Stahl hergestellt sein.

Die Rundschienen können insbesondere ohne Abdeckung und ohne Antriebselemente im Boden integriert werden und dabei ein Überfahren mit Patientenbetten und Instrumententischen ermöglichen. Die Antriebskraft für die Translationsbewegung der Computertomographie-Gantry kann beispielsweise basierend auf einem Kraftschluss, insbesondere Reibschluss, zwischen den Rädern des Satzes von Rädern und den Schienen des Satzes von Schienen von dem Fahrwagen auf das Schienensystem übertragen werden.

Eine Ausführungsform sieht vor, dass der Fahrwagen für jedes Rad des Satzes von Rädern einen Rad-Direktantrieb, der mit diesem Rad zusammenwirkt und anteilig zu der Antriebskraft für die Translationsbewegung der Computertomographie-Gantry beiträgt, aufweist. Insbesondere kann vorgesehen sein, dass für jedes Rad des Satzes von Rädern der Rad-Direktantrieb, der mit diesem Rad zusammenwirkt, dieses Rad direkt antreibt und dadurch anteilig zu der Antriebskraft für die Translationsbewegung der Computertomographie-Gantry beiträgt. Insbesondere kann vorgesehen sein, dass die Antriebskraft für die Translationsbewegung der Computertomographie-Gantry von den Rad-Direktantrieben der Räder des Satzes von Rädern zusammen erzeugt wird. Der Rad-Direktantrieb kann beispielsweise einen Elektromotor, insbesondere einen Elektro-Radnabenmotor, aufweisen.

Eine Ausführungsform sieht vor, dass das medizinische Bildgebungssystem ferner ein Positionsmesssystem aufweist und dass das Positionsmesssystem zum Generieren einer Positionsinformation eingerichtet ist, wobei die Positionsinformation eine Position der Computertomographie-Gantry entlang des Schienensystems betrifft.

Die Position der Computertomographie-Gantry entlang des Schienensystems kann insbesondere relativ zu einem Bezugspunkt, der relativ zu der Grundfläche und/oder relativ zu dem Schienensystem ruht, insbesondere während der Translationsbewegung der Computertomographie-Gantry relativ zu der Grundfläche und/oder relativ zu dem Schienensystem ruht, definiert sein. Insbesondere kann vorgesehen sein, dass die Position der Computertomographie-Gantry entlang des Schienensystems während der Translationsbewegung der Computertomographie-Gantry kontinuierlich, insbesondere mit einer ausreichend hohen Abtastrate, gemessen wird, insbesondere derart gemessen wird, dass die Positionsinformation für jeden Projektionsdatensatz, der mittels der Computertomographie-Gantry von einem Untersuchungsobjekt während der Translationsbewegung der Computertomographie-Gantry aufgenommen wurde, eine Position der Computertomographie-Gantry, an der dieser Projektionsdatensatz aufgenommen wurde, umfasst.

Eine Ausführungsform sieht vor, dass das Positionsmesssystem zum Generieren der Positionsinformation basierend auf einer Messung, insbesondere basierend auf einer berührungslosen Messung, der Position der Computertomographie-Gantry entlang des Schienensystems eingerichtet ist.

Die berührungslose Messung kann beispielsweise optisch, magnetisch, magnetostriktiv, induktiv und/oder kapazitiv erfolgen und/oder auf einer Laufzeitmessung basieren. Die Kombination aus kraftschlüssiger Antriebskraftübertragung zwischen Rad und Schiene einerseits sowie der berührungslosen Positionsmessung andererseits ermöglicht eine genaue Positionierung und Positionserkennung des Fahrwagens bei minimaler Eingriffsfläche, wobei sowohl beim Antrieb als auch bei der Positionsmessung auf einen Formschluss verzichtet werden kann. Dadurch wird die Reinigbarkeit verbessert und der Verschleiß reduziert.

Die Messung der Position der Computertomographie-Gantry entlang des Schienensystems kann insbesondere absolut, beispielsweise mittels eines Absolutwertgebers, oder inkremental, beispielsweise mittels eines Inkrementalgebers, erfolgen.

Eine Ausführungsform sieht vor, dass das Positionsmesssystem eine Maßspur und einen Positionssensor aufweist, wobei die Maßspur relativ zu dem Schienensystem ruht und sich entlang des Schienensystems erstreckt, wobei der Positionssensor derart mit dem Fahrwagen verbunden ist, dass er der Translationsbewegung der Computertomographie-Gantry folgt und während der Translationsbewegung der Computertomographie-Gantry mit der Maßspur zusammenwirkt, insbesondere um die Messung, insbesondere die berührungslose Messung, der Position der Computertomographie-Gantry entlang des Schienensystems auszuführen.

Die Maßspur kann beispielsweise eine Codespur sein. Der Positionssensor kann insbesondere zum Abtasten der Codespur eingerichtet sein. Die Maßspur kann beispielsweise ein Magnetband sein. Das Magnetband kann insbesondere in regelmäßigen Abständen magnetisiert sein. Die Maßspur kann beispielsweise ein Maßband, insbesondere ein Edelstahl-Maßband sein, und/oder relativ zu der Grundfläche fest verankert sein.

Eine Ausführungsform sieht vor, dass das medizinische Bildgebungssystem ferner ein Tragprofil aufweist und dass das Tragprofil eine Maßspur-Nut zur Aufnahme, insbesondere zur formschlüssigen Aufnahme, der Maßspur aufweist und die Maßspur in die Maßspur-Nut aufgenommen ist, insbesondere formschlüssig aufgenommen ist, wobei das Tragprofil eine Schienen-Nut zur formschlüssigen Aufnahme einer Schiene des Schienensystems aufweist und die Schiene des Schienensystems in die Schienen-Nut formschlüssig aufgenommen ist.

Insbesondere kann vorgesehen sein, dass sich das Tragprofil entlang des Schienensystems erstreckt und/oder dass das Tragprofil relativ zu der Grundfläche fest verankert ist. Insbesondere können die Maßspur-Nut und die Schienen-Nut im Wesentlichen parallel zueinander angeordnet sein. Insbesondere kann die Maßspur mit dem Tragprofil verklebt sein.

Eine Ausführungsform sieht vor, dass das medizinische Bildgebungssystem ferner eine Datenverarbeitungseinheit aufweist und dass die Datenverarbeitungseinheit dazu eingerichtet ist, ein Antriebssignal basierend auf der Positionsinformation zu berechnen, wobei der Fahrwagen einen Fahrantrieb aufweist, wobei der Fahrantrieb dazu eingerichtet ist, die Antriebskraft für die Translationsbewegung der Computertomographie-Gantry in Abhängigkeit von dem Antriebssignal zu erzeugen.

Insbesondere kann vorgesehen sein, dass die Rad-Direktantriebe der Räder des Satzes von Rädern zusammen den Fahrantrieb bilden.

Hiermit ist ferner ein medizinisches System offenbart, aufweisend eine Tragkonstruktion, einen Fahrwagen und ein Schienensystem und ein Tragprofil,
- wobei die Tragkonstruktion mittels des Fahrwagens und des Schienensystems derart bewegbar gelagert ist, dass entlang des Schienensystems eine Translationsbewegung der Tragkonstruktion relativ zu einer Grundfläche ausführbar ist,
- wobei das Tragprofil in eine Vertiefung, die relativ zu der Grundfläche ausgebildet ist, formschlüssig aufgenommen ist,
- wobei das Tragprofil eine Schienen-Nut zur formschlüssigen Aufnahme einer Schiene des Schienensystems aufweist,
- wobei die Schiene des Schienensystems in die Schienen-Nut derart formschlüssig aufgenommen ist, dass die Schiene des Schienensystems in Bezug auf eine vertikale Richtung nicht über die Grundfläche hinausragt.

Hiermit ist ferner ein medizinisches System offenbart, aufweisend eine Tragkonstruktion, einen Fahrwagen, ein Schienensystem und ein Tragprofil,
- wobei die Tragkonstruktion mittels des Fahrwagens und des Schienensystems derart bewegbar gelagert ist, dass entlang des Schienensystems eine Translationsbewegung der Tragkonstruktion relativ zu einer Grundfläche ausführbar ist,
- wobei das Tragprofil in eine Vertiefung, die relativ zu der Grundfläche ausgebildet ist, formschlüssig aufgenommen ist,
- wobei das Tragprofil eine Schienen-Nut zur formschlüssigen Aufnahme einer Schiene des Schienensystems aufweist,
- wobei das Tragprofil im Bereich der Schienen-Nut ein profilseitiges Verbindungselement aufweist,
- wobei die Schiene des Schienensystems ein schienenseitiges Verbindungselement aufweist, das zu dem profilseitigen Verbindungselement korrespondierend ausgebildet ist,
- wobei die Schiene des Schienensystems durch einen Formschluss des profilseitigen Verbindungselements und des schienenseitigen Verbindungselements gegen eine Drehung um eine Längsachse der Schiene des Schienensystems relativ zu dem Tragprofil gesichert ist.

Optional ist vorgesehen, dass der Fahrwagen und das Schienensystem dazu eingerichtet sind, eine Antriebskraft für die Translationsbewegung der Tragkonstruktion kraftschlüssig von dem Fahrwagen auf das Schienensystem zu übertragen.

Das medizinische System mit der Tragkonstruktion kann beispielsweise in Analogie zu einem der Aspekte, die für das medizinische Bildgebungssystem mit der Computertomographie-Gantry beschrieben sind, ausgebildet sein. Das medizinische System kann beispielsweise ein Röntgenbildgebungssystem, insbesondere mit einem C-Bogen-Arm als Tragkonstruktion, ein Magnetresonanzbildgebungssystem, insbesondere mit einer die Körperspule haltenden Tragkonstruktion, ein Strahlentherapiegerät, insbesondere mit einer die Strahlenquelle haltenden Tragkonstruktion, oder eine Patientenlagerungsvorrichtung, insbesondere mit einem Patientenbett als Tragkonstruktion, sein.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Die Verwendung des unbestimmten Artikels "ein" bzw. "eine" schließt nicht aus, dass das betroffene Merkmal auch mehrfach vorhanden sein kann.

Im Folgenden werden Merkmale der Erfindung anhand von Beispielen unter Hinweis auf die beigefügten Figuren erläutert. Die Darstellung in den Figuren ist schematisch, stark vereinfacht und nicht zwingend maßstabsgetreu.

Die Fig. 1 zeigt ein Tragprofil, eine Schiene und eine Maßspur in einer ersten Ansicht.

Die Fig. 2 zeigt das Tragprofil, die Schiene und die Maßspur in einer zweiten Ansicht.

Die Fig. 3 zeigt ein medizinisches Bildgebungssystem mit einer Computertomographie-Gantry, einem Fahrwagen und einem Schienensystem.

Die Fig. 4 zeigt ein Tragprofil und eine Schiene mit einer Verdrehsicherung.

Die Fig. 5 zeigt ein Ablaufdiagramm eines Verfahrens zum Bewegen einer Computertomographie-Gantry.

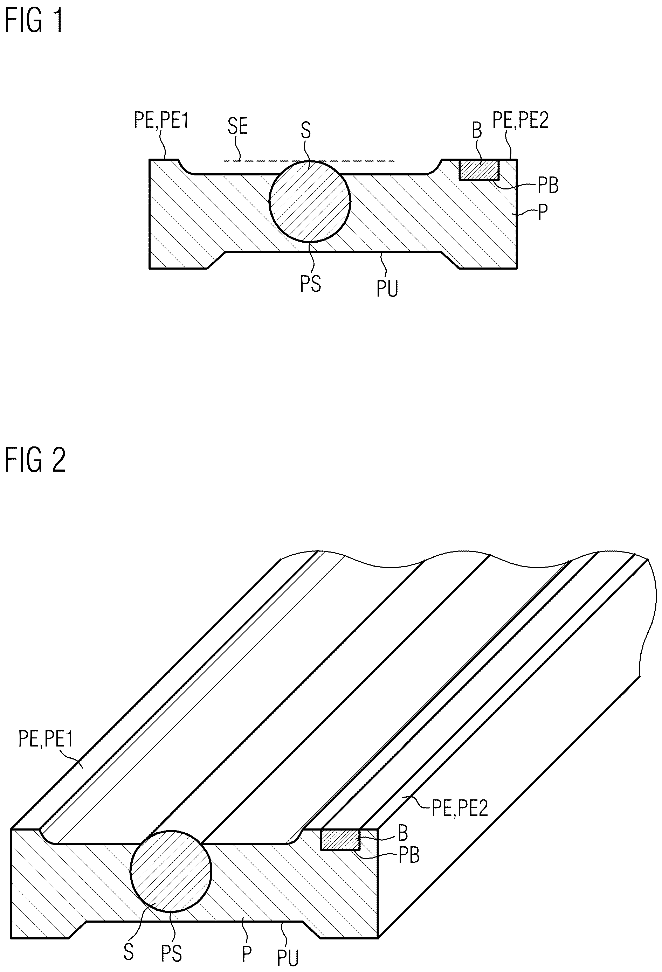

Die Fig. 1 zeigt das Tragprofil P, die Schiene S und die Maßspur B in einer ersten Ansicht, wobei das Tragprofil P eine Maßspur-Nut PB zur formschlüssigen Aufnahme der Maßspur B aufweist und die Maßspur B in die Maßspur-Nut PB formschlüssig aufgenommen ist, wobei das Tragprofil P eine Schienen-Nut PS zur formschlüssigen Aufnahme einer Schiene S des Schienensystems L aufweist und die Schiene S des Schienensystems L in die Schienen-Nut PS formschlüssig aufgenommen ist. Das Tragprofil P weist die Verankerungsstruktur PU zur formschlüssigen Verankerung in einer entsprechenden Aussparung der Grundfläche U auf. Die Fig. 2 zeigt das Tragprofil P, die Schiene S und die Maßspur B in einer zweiten Ansicht.

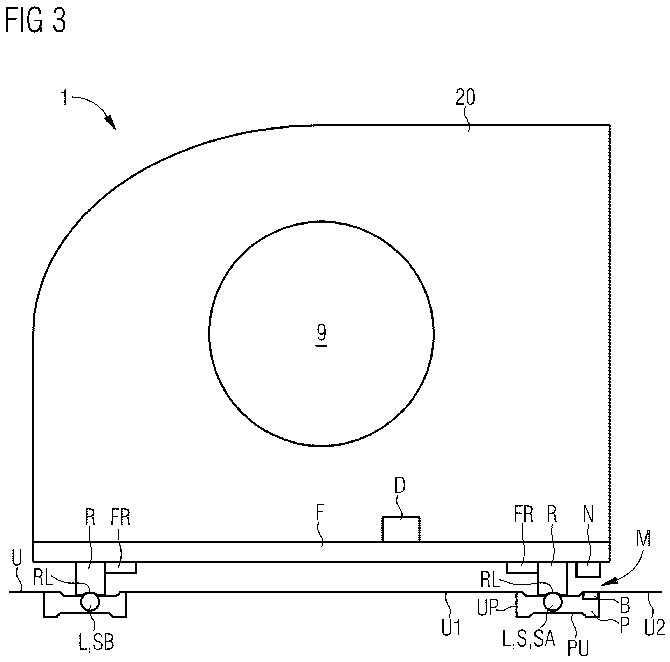

Die Fig. 3 zeigt das medizinische Bildgebungssystem 1, aufweisend die Computertomographie-Gantry 20, den Fahrwagen F und das Schienensystem L, wobei die Computertomographie-Gantry 20 mittels des Fahrwagens F und des Schienensystems L derart bewegbar gelagert ist, dass entlang des Schienensystems L eine Translationsbewegung der Computertomographie-Gantry 20 ausführbar ist, wobei der Fahrwagen F und das Schienensystem L dazu eingerichtet sind, eine Antriebskraft für die Translationsbewegung der Computertomographie-Gantry 20 kraftschlüssig von dem Fahrwagen F auf das Schienensystem L zu übertragen.

Zwischen dem Fahrwagen F und dem Schienensystem L ist ein Satz von Rad-Schiene-Rollkontakten RL ausgebildet, wobei der Fahrwagen F und das Schienensystem L dazu eingerichtet sind, die Antriebskraft für die Translationsbewegung der Computertomographie-Gantry 20 mittels des Satzes von Rad-Schiene-Rollkontakten RL kraftschlüssig von dem Fahrwagen F auf das Schienensystem L zu übertragen. Der Satz von Rad-Schiene-Rollkontakten RL nimmt die gesamte Gewichtskraft des Fahrwagens F und der Computertomographie-Gantry 20 auf, wobei jeder Rad-Schiene-Rollkontakt, welcher in dem Satz von Rad-Schiene-Rollkontakten RL enthalten ist und zumindest einen Teil der gesamten Gewichtskraft des Fahrwagens F und der Computertomographie-Gantry 20 aufnimmt, zumindest einen Teil der Antriebskraft für die Translationsbewegung der Computertomographie-Gantry 20 kraftschlüssig überträgt. Das Schienensystem L weist einen Satz von Schienen auf, wobei der Fahrwagen F einen Satz von Rädern R aufweist, wobei der Satz von Rädern R auf dem Satz von Schienen rollend angeordnet ist. Der Fahrwagen F weist für jedes Rad des Satzes von Rädern R einen Rad-Direktantrieb, der mit diesem Rad zusammenwirkt und anteilig zu der Antriebskraft für die Translationsbewegung der Computertomographie-Gantry 20 beiträgt, auf.

Das medizinische Bildgebungssystem 1 weist ferner das Positionsmesssystem M auf, wobei das Positionsmesssystem M zum Generieren S2 einer Positionsinformation eingerichtet ist, wobei die Positionsinformation eine Position der Computertomographie-Gantry 20 entlang des Schienensystems L betrifft. Das Positionsmesssystem M ist zum Generieren S2 der Positionsinformation basierend auf einer berührungslosen Messung der Position der Computertomographie-Gantry 20 entlang des Schienensystems L eingerichtet. Das Positionsmesssystem M weist die Maßspur B und den Positionssensor N auf, wobei die Maßspur B relativ zu dem Schienensystem L ruht und sich entlang des Schienensystems L erstreckt, wobei der Positionssensor N derart mit dem Fahrwagen F verbunden ist, dass er der Translationsbewegung der Computertomographie-Gantry 20 folgt und während der Translationsbewegung der Computertomographie-Gantry 20 mit der Maßspur B zusammenwirkt. Beispielsweise kann auch im Bereich der anderen Schiene des Schienensystems L (im linken Teil der Fig. 3) ein entsprechendes Positionsmesssystem, insbesondere mit Maßspur und Positionssensor, vorgesehen sein.

Das medizinische Bildgebungssystem 1 weist ferner eine Datenverarbeitungseinheit D auf, wobei die Datenverarbeitungseinheit D dazu eingerichtet ist, ein Antriebssignal basierend auf der Positionsinformation zu berechnen, wobei der Fahrwagen F einen Fahrantrieb FR aufweist, wobei der Fahrantrieb FR dazu eingerichtet ist, die Antriebskraft für die Translationsbewegung der Computertomographie-Gantry 20 in Abhängigkeit von dem Antriebssignal zu erzeugen.

Die Computertomographie-Gantry 20 weist die Öffnung 9 auf. Durch die Translationsbewegung der Computertomographie-Gantry 20 kann ein Untersuchungstisch in die Öffnung 9 eingeführt werden, insbesondere zusammen mit einem auf dem Untersuchungstisch gelagerten Untersuchungsobjekt in die Öffnung 9 eingeführt werden.

Das gezeigte Beispiel betrifft ein medizinisches Bildgebungssystem 1, aufweisend eine Computertomographie-Gantry 20, einen Fahrwagen F, ein Schienensystem L und ein Tragprofil P,
- wobei die Computertomographie-Gantry 20 mittels des Fahrwagens F und des Schienensystems L derart bewegbar gelagert ist, dass entlang des Schienensystems L eine Translationsbewegung der Computertomographie-Gantry 20 relativ zu einer Grundfläche U ausführbar ist,
- wobei das Tragprofil P in eine Vertiefung UP, die relativ zu der Grundfläche U ausgebildet ist, formschlüssig aufgenommen ist,
- wobei das Tragprofil P eine Schienen-Nut PS zur formschlüssigen Aufnahme einer Schiene S des Schienensystems L aufweist,
- wobei die Schiene S des Schienensystems L in die Schienen-Nut PS derart formschlüssig aufgenommen ist, dass die Schiene S des Schienensystems L in Bezug auf eine vertikale Richtung nicht über die Grundfläche U hinausragt.

Das gezeigte Beispiel sieht vor, dass jede zu der Grundfläche U parallele Tangentialebene SE an die Schiene S des Schienensystems L nicht oberhalb der Grundfläche U verläuft.

Das gezeigte Beispiel sieht vor, dass die Grundfläche U einen ersten Grundflächenbereich U1 und einen zweiten Grundflächenbereich U2 aufweist,
- wobei der erste Grundflächenbereich U1 und der zweite Grundflächenbereich U2 zueinander komplanar sind,
- wobei sich die Vertiefung UP zwischen dem ersten Grundflächenbereich U1 und dem zweiten Grundflächenbereich U2 befindet,
- wobei die Schiene S des Schienensystems L in die Schienen-Nut PS derart formschlüssig aufgenommen ist, dass die Schiene S des Schienensystems L nicht über den ersten Grundflächenbereich U1 hinausragt und dass die Schiene S des Schienensystems L nicht über den zweiten Grundflächenbereich U2 hinausragt.

Das gezeigte Beispiel sieht vor, dass das Tragprofil P eine Anschlussfläche PE aufweist,
- wobei das Tragprofil P in die Vertiefung UP derart formschlüssig aufgenommen ist, dass die Anschlussfläche PE stufenlos, insbesondere komplanar, an die Grundfläche U, insbesondere als eine Fortsetzung der Grundfläche U, angefügt ist.

Das gezeigte Beispiel sieht vor, dass die Anschlussfläche PE einen ersten Anschlussflächenbereich PE1 und einen zweiten Anschlussflächenbereich PE2 aufweist,
- wobei sich die Schiene S des Schienensystems L zwischen dem ersten Anschlussflächenbereich PE1 und dem zweiten Anschlussflächenbereich PE2 befindet,
- wobei das Tragprofil P in die Vertiefung UP derart formschlüssig aufgenommen ist, dass der erste Anschlussflächenbereich PE1 stufenlos, insbesondere komplanar, an den ersten Grundflächenbereich U1, insbesondere als eine Fortsetzung des ersten Grundflächenbereichs U1, angefügt ist und dass der zweite Anschlussflächenbereich PE2 stufenlos, insbesondere komplanar, an den zweiten Grundflächenbereich U2, insbesondere als eine Fortsetzung des zweiten Grundflächenbereichs U2, angefügt ist.

Das gezeigte Beispiel sieht vor, dass sich die Grundfläche U im Wesentlichen horizontal erstreckt,
- wobei die Schiene S des Schienensystems L in die Schienen-Nut PS derart formschlüssig aufgenommen ist, dass die Schiene S des Schienensystems L in Bezug auf die vertikale Richtung nicht über die Grundfläche U hinausragt.

Das gezeigte Beispiel sieht vor, dass die Schiene S des Schienensystems L eine erste Schiene SA des Schienensystems L ist und parallel zu einer zweiten Schiene SB des Schienensystems L angeordnet ist,
- wobei die Grundfläche U im Wesentlichen parallel zu einer Schienenebene ist, wobei die Schienenebene durch die erste Schiene SA des Schienensystems L und durch die zweite Schiene SB des Schienensystems L verläuft.

Das gezeigte Beispiel sieht vor, dass die Schiene S des Schienensystems L in einer Querschnittsebene, die zu einer Längsrichtung der Schiene S des Schienensystems L senkrecht ist, ein Rundprofil, insbesondere ein kreisrundes Profil, aufweist.

Das gezeigte Beispiel sieht vor, dass das Tragprofil P im Bereich der Schienen-Nut PS ein profilseitiges Verbindungselement T aufweist,
- wobei die Schiene S des Schienensystems L ein schienenseitiges Verbindungselement ST aufweist, das zu dem profilseitigen Verbindungselement T korrespondierend ausgebildet ist,
- wobei die Schiene S des Schienensystems L durch einen Formschluss des profilseitigen Verbindungselements T und des schienenseitigen Verbindungselements ST gegen eine Drehung um eine Längsachse der Schiene S des Schienensystems L relativ zu dem Tragprofil P gesichert ist.

Das gezeigte Beispiel sieht vor, dass das profilseitige Verbindungselement T stiftförmig in einer Richtung, die zu der Längsachse der Schiene S des Schienensystems L senkrecht ist, zu der Schiene S des Schienensystems L hin ragt,
- wobei das schienenseitige Verbindungselement ST eine Aussparung zur Aufnahme des profilseitigen Verbindungselements T aufweist.

Das gezeigte Beispiel sieht vor, dass die Aussparung eine Bohrung, insbesondere in Form eines Kreislochs oder Langlochs, ist und/oder dass sich die Aussparung parallel zu der Längsachse der Schiene S des Schienensystems L länglich erstreckt.

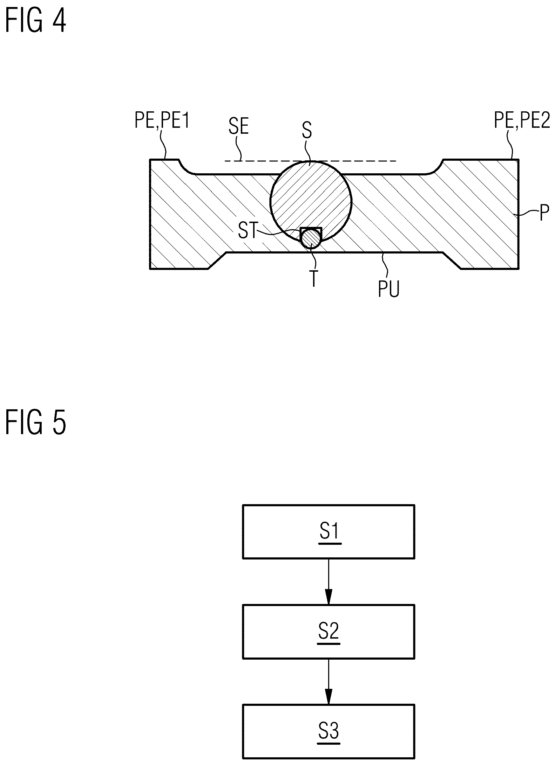

Die Fig. 5 zeigt ein Ablaufdiagramm eines Verfahrens zum Bewegen einer Computertomographie-Gantry 20, wobei die Computertomographie-Gantry 20 mittels eines Fahrwagens F und eines Schienensystems L derart bewegbar gelagert ist, dass entlang des Schienensystems L eine Translationsbewegung der Computertomographie-Gantry 20 ausführbar ist, das Verfahren umfassend:
- ein Ausführen S1 der Translationsbewegung der Computertomographie-Gantry 20 entlang des Schienensystems L, wobei eine Antriebskraft für die Translationsbewegung der Computertomographie-Gantry 20 kraftschlüssig von dem Fahrwagen F auf das Schienensystem L übertragen wird,
- ein Generieren S2 einer Positionsinformation mittels eines Positionsmesssystems M während des Ausführens S1 der Translationsbewegung der Computertomographie-Gantry 20 entlang des Schienensystems L, wobei die Positionsinformation eine Position der Computertomographie-Gantry 20 entlang des Schienensystems L betrifft,
- ein Bereitstellen S3 der Positionsinformation.

## Patentansprüche

1. Medizinisches Bildgebungssystem (1), aufweisend eine Computertomographie-Gantry (20), einen Fahrwagen (F), ein Schienensystem (L) und ein Tragprofil (P),
- wobei die Computertomographie-Gantry (20) mittels des Fahrwagens (F) und des Schienensystems (L) derart bewegbar gelagert ist, dass entlang des Schienensystems (L) eine Translationsbewegung der Computertomographie-Gantry (20) relativ zu einer Grundfläche (U) ausführbar ist,
- wobei das Tragprofil (P) in eine Vertiefung (UP), die relativ zu der Grundfläche (U) ausgebildet ist, formschlüssig aufgenommen ist,
- wobei das Tragprofil (P) eine Schienen-Nut (PS) zur formschlüssigen Aufnahme einer Schiene (S) des Schienensystems (L) aufweist,
- wobei die Schiene (S) des Schienensystems (L) in die Schienen-Nut (PS) derart formschlüssig aufgenommen ist, dass die Schiene (S) des Schienensystems (L) in Bezug auf eine vertikale Richtung nicht über die Grundfläche (U) hinausragt.

2. Medizinisches Bildgebungssystem (1) nach Anspruch 1,
- wobei jede zu der Grundfläche (U) parallele Tangentialebene (SE) an die Schiene (S) des Schienensystems (L) nicht oberhalb der Grundfläche (U) verläuft.

3. Medizinisches Bildgebungssystem (1) nach Anspruch 1 oder 2,
- wobei die Grundfläche (U) einen ersten Grundflächenbereich (U1) und einen zweiten Grundflächenbereich (U2) aufweist,
- wobei der erste Grundflächenbereich (U1) und der zweite Grundflächenbereich (U2) zueinander komplanar sind,
- wobei sich die Vertiefung (UP) zwischen dem ersten Grundflächenbereich (U1) und dem zweiten Grundflächenbereich (U2) befindet,
- wobei die Schiene (S) des Schienensystems (L) in die Schienen-Nut (PS) derart formschlüssig aufgenommen ist, dass die Schiene (S) des Schienensystems (L) nicht über den ersten Grundflächenbereich (U1) hinausragt und dass die Schiene (S) des Schienensystems (L) nicht über den zweiten Grundflächenbereich (U2) hinausragt.

4. Medizinisches Bildgebungssystem (1) nach einem der Ansprüche 1 bis 3,
- wobei das Tragprofil (P) eine Anschlussfläche (PE) aufweist,
- wobei das Tragprofil (P) in die Vertiefung (UP) derart formschlüssig aufgenommen ist, dass die Anschlussfläche (PE) stufenlos an die Grundfläche (U) angefügt ist.

5. Medizinisches Bildgebungssystem (1) nach den Ansprüchen 3 und 4,
- wobei die Anschlussfläche (PE) einen ersten Anschlussflächenbereich (PE1) und einen zweiten Anschlussflächenbereich (PE2) aufweist,
- wobei sich die Schiene (S) des Schienensystems (L) zwischen dem ersten Anschlussflächenbereich (PE1) und dem zweiten Anschlussflächenbereich (PE2) befindet,
- wobei das Tragprofil (P) in die Vertiefung (UP) derart formschlüssig aufgenommen ist, dass der erste Anschlussflächenbereich (PE1) stufenlos an den ersten Grundflächenbereich (U1) angefügt ist und dass der zweite Anschlussflächenbereich (PE2) stufenlos an den zweiten Grundflächenbereich (U2) angefügt ist.

6. Medizinisches Bildgebungssystem (1) nach einem der Ansprüche 1 bis 5,
- wobei sich die Grundfläche (U) im Wesentlichen horizontal erstreckt,
- wobei die Schiene (S) des Schienensystems (L) in die Schienen-Nut (PS) derart formschlüssig aufgenommen ist, dass die Schiene (S) des Schienensystems (L) in Bezug auf die vertikale Richtung nicht über die Grundfläche (U) hinausragt.

7. Medizinisches Bildgebungssystem (1) nach einem der Ansprüche 1 bis 6,
- wobei die Schiene (S) des Schienensystems (L) eine erste Schiene (SA) des Schienensystems (L) ist und parallel zu einer zweiten Schiene (SB) des Schienensystems (L) angeordnet ist,
- wobei die Grundfläche (U) im Wesentlichen parallel zu einer Schienenebene ist, wobei die Schienenebene durch die erste Schiene (SA) des Schienensystems (L) und durch die zweite Schiene (SB) des Schienensystems (L) verläuft.

8. Medizinisches Bildgebungssystem (1) nach einem der Ansprüche 1 bis 7,
- wobei die Schiene (S) des Schienensystems (L) in einer Querschnittsebene, die zu einer Längsrichtung der Schiene (S) des Schienensystems (L) senkrecht ist, ein Rundprofil aufweist.

9. Medizinisches Bildgebungssystem (1) nach einem der Ansprüche 1 bis 7,
- wobei die Schiene (S) des Schienensystems (L) in einer Querschnittsebene, die zu einer Längsrichtung der Schiene (S) des Schienensystems (L) senkrecht ist, ein verrundetes Rechteckprofil aufweist.

10. Medizinisches Bildgebungssystem (1) nach einem der Ansprüche 1 bis 9,
- wobei das Tragprofil (P) im Bereich der Schienen-Nut (PS) ein profilseitiges Verbindungselement (T) aufweist,
- wobei die Schiene (S) des Schienensystems (L) ein schienenseitiges Verbindungselement (ST) aufweist, das zu dem profilseitigen Verbindungselement (T) korrespondierend ausgebildet ist,
- wobei die Schiene (S) des Schienensystems (L) durch einen Formschluss des profilseitigen Verbindungselements (T) und des schienenseitigen Verbindungselements (ST) gegen eine Drehung um eine Längsachse der Schiene (S) des Schienensystems (L) relativ zu dem Tragprofil (P) gesichert ist.

11. Medizinisches Bildgebungssystem (1), aufweisend eine Computertomographie-Gantry (20), einen Fahrwagen (F), ein Schienensystem (L) und ein Tragprofil (P),
- wobei die Computertomographie-Gantry (20) mittels des Fahrwagens (F) und des Schienensystems (L) derart bewegbar gelagert ist, dass entlang des Schienensystems (L) eine Translationsbewegung der Computertomographie-Gantry (20) relativ zu einer Grundfläche (U) ausführbar ist,
- wobei das Tragprofil (P) in eine Vertiefung (UP), die relativ zu der Grundfläche (U) ausgebildet ist, formschlüssig aufgenommen ist,
- wobei das Tragprofil (P) eine Schienen-Nut (PS) zur formschlüssigen Aufnahme einer Schiene (S) des Schienensystems (L) aufweist,
- wobei das Tragprofil (P) im Bereich der Schienen-Nut (PS) ein profilseitiges Verbindungselement (T) aufweist,
- wobei die Schiene (S) des Schienensystems (L) ein schienenseitiges Verbindungselement (ST) aufweist, das zu dem profilseitigen Verbindungselement (T) korrespondierend ausgebildet ist,
- wobei die Schiene (S) des Schienensystems (L) durch einen Formschluss des profilseitigen Verbindungselements (T) und des schienenseitigen Verbindungselements (ST) gegen eine Drehung um eine Längsachse der Schiene (S) des Schienensystems (L) relativ zu dem Tragprofil (P) gesichert ist.

12. Medizinisches Bildgebungssystem (1) nach Anspruch 10 oder 11,
- wobei das profilseitige Verbindungselement (T) stiftförmig in einer Richtung, die zu der Längsachse der Schiene (S) des Schienensystems (L) senkrecht ist, zu der Schiene (S) des Schienensystems (L) hin ragt,
- wobei das schienenseitige Verbindungselement (ST) eine Aussparung zur Aufnahme des profilseitigen Verbindungselements (T) aufweist.

13. Medizinisches Bildgebungssystem (1) nach Anspruch 10 oder 11,
- wobei das schienenseitige Verbindungselement (ST) stiftförmig in einer Richtung, die zu der Längsachse der Schiene (S) des Schienensystems (L) senkrecht ist, von der Schiene (S) des Schienensystems (L) weg ragt,
- wobei das profilseitige Verbindungselement (T) eine Aussparung zur Aufnahme des schienenseitigen Verbindungselements (T) aufweist.

14. Medizinisches Bildgebungssystem (1) nach Anspruch 12 oder 13,
- wobei die Aussparung eine Bohrung, insbesondere in Form eines Kreislochs, ist.

15. Medizinisches Bildgebungssystem (1) nach Anspruch 12 oder 13,
- wobei die Aussparung ein Langloch ist und/oder sich parallel zu der Längsachse der Schiene (S) des Schienensystems (L) länglich erstreckt.
